(19) Europäisches Patentamt
European Patent Office
Office européen des brevets

(11) **EP 1 545 634 B1**

(12) **EUROPEAN PATENT SPECIFICATION**

(45) Date of publication and mention
of the grant of the patent:
**01.08.2012 Bulletin 2012/31**

(21) Application number: **03793373.6**

(22) Date of filing: **21.08.2003**

(51) Int Cl.:
*A61K 9/72* (2006.01)     *A61K 31/167* (2006.01)
*A61K 31/58* (2006.01)

(86) International application number:
**PCT/US2003/026542**

(87) International publication number:
**WO 2004/017918 (04.03.2004 Gazette 2004/10)**

(54) **METHOD OF PREPARING DRY POWDER INHALATION COMPOSITIONS**

VERFAHREN ZUR HERSTELLUNG VON TROCKENPULVER-ZUSAMMENSETZUNGEN ZUR INHALATION

PROCEDE DE PREPARATION DE COMPOSITIONS POUR INHALATION A BASE DE POUDRES SECHES

(84) Designated Contracting States:
**AT BE BG CH CY CZ DE DK EE ES FI FR GB GR HU IE IT LI LU MC NL PT RO SE SI SK TR**
Designated Extension States:
**MK**

(30) Priority: **21.08.2002 GB 0219511**

(43) Date of publication of application:
**29.06.2005 Bulletin 2005/26**

(60) Divisional application:
**11008728.5**

(73) Proprietor: **NORTON HEALTHCARE LIMITED**
**Swiss Cottage**
**London NW6 3RZ (GB)**

(72) Inventor: **ZENG, Xian-Ming**
**Surrey CR4 2HS (GB)**

(74) Representative: **Cottam, David William et al**
**Teva Europe Patent Department**
**167 Fleet Street**
**London**
**EC4A 2EA (GB)**

(56) References cited:
WO-A-01/70198     WO-A-01/78737
WO-A-01/89492     WO-A-99/64014
US-A- 5 972 919   US-A- 5 985 248
US-A- 6 103 270

**Description**

TECHNICAL FIELD OF THE INVENTION

The invention relates to methods of preparing dry powder inhalation compositions, compositions and uses of the same. The compositions of the invention are characterized by dose uniformity, reliability and dispersion of medicaments uniformity.

## BACKGROUND OF THE INVENTION

The preparation of ternary mixtures of a particulate carrier, a first particulate inhalant medicament and a second particulate inhalant medicament poses particular problems when one medicament is present at a relatively small proportion compared to the other medicament. It is difficult to prepare mixtures which are homogeneous. In addition, small quantities of medicament may sometimes bind to the inert carrier, which can affect the amount of medicament that is made available to the patient when the formulation is delivered, *e.g.* by means of a dry powder inhaler (DPI) device. In such devices, a metered dose of composition comprising one or more active ingredients and an inert carrier, such as lactose, is dispensed into the air stream that is produced by the inspirational effort of the patient. The medicaments and carrier are entrained in this air stream, with only the fine particles of medicament entering the deep recesses of the lung (which is the site of action of the medicament), the inert excipient being deposited either in the mouth of in the upper region of the lungs.

The accurate metering of highly potent inhalant drugs causes particular problems, as the quantity of medicament in the composition relative to that of the carrier is often particularly small (less than 1 part of drug to 50 parts of carrier). This is exemplified by the medicament formoterol, which is often administered to patients at a dose of less than 60 micrograms (doses may be as small as 6 micrograms). WO 01/70198 describes a process for the manufacture of a dry powder inhalation composition wherein the non - polar drug is mixed with the polar excipient before adding the polar drug.

Thus, methods of producing ternary mixtures that are homogeneous and can be used with suitable dry powder inhalers, to give dose uniformity, reliability, and uniform dispersion of a plurality of medicaments in the composition are needed.

## SUMMARY OF THE INVENTION

It has been discovered that dry powder preparations characterised by dose uniformity, reliability and dispersion of medicaments uniformity may be obtained by mixing specified ratios of medicaments to carrier in a specified manner as described herein. Thus, the invention provides a method of preparing a dry powder inhalation composition according to claim 1. Accordingly, in an embodiment, the invention provides a method of preparing a dry powder inhalation composition comprising a pharmaceutically acceptable particulate carrier, a first particulate inhalant medicament and a second particulate inhalant medicament, where the proportion of the second medicament to the carrier is smaller relative to the proportion of the first medicament to the carrier. The method is characterised in thai the carrier is mixed with a first portion of the first particulate inhalant medicament, the resulting first mixture is mixed with substantially all of the second particulate inhalant medicament to give a mixture. The remaining portion of the first particulate inhalant medicament is mixed with the second mixture to give the desired dry powder inhalation composition.

## DETAILED DESCRIPTION OF THE INVENTION

The invention provides a method of preparing a dry powder inhalation composition comprising a carrier, and first and second particulate medicament according to claim 1. The method is characterised in that the carrier is mixed with a first portion of the first medicament, the resulting mixture is mixed with substantially all of the second medicament to give a pre-mixture and then the remaining portion of the first medicament is mixed with the pre-mixture to give the desired dry powder inhalation composition. Also provided are dry powder compositions and methods of using them with a dry powder inhalation device.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001).

Any suitable materials and/or methods known to those of skill can be utilized in carrying out the present invention. However, preferred materials and methods are described. Materials, reagents and the like to which reference is made in the following description and examples are obtainable from commercial sources, unless otherwise noted.

As used in this specification, whether in a transitional phrase or in the body of the claim, the terms "comprise (s)" and "comprising" are to be interpreted as having an openended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used in this specification, the singular forms "a," "an" and "the" specifically also encompass the plural forms of the terms to which they refer, unless the content clearly dictates otherwise.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

As used herein, unless specifically indicated otherwise, the word "or" is used in the "inclusive" sense of "and/or" and not the "exclusive" sense of "either/or."

Reference is made hereinafter in detail to specific embodiments of the invention. While the invention will be described in conjunction with these specific embodiments, it will be understood that it is not intended to limit the invention to such specific embodiments. On the contrary, it is intended to cover alternatives, modifications, and equivalents as may be included within the scope of the invention as defined by the appended claims. In the following description, numerous specific details are set forth in order to provide a thorough understanding of the present invention. The present invention may be practiced without some or all of these specific details. In other instances, well known process operations have not been described in detail, in order not to unnecessarily obscure the present invention.

An aspect of the invention provides a method for preparing a dry powder inhalation composition comprising the steps of mixing a first portion of a first particulate inhalant medicament with the carrier to form an first mixture; thereafter mixing the first mixture with a second particulate inhalant medicament to form a second mixture; and mixing the second mixture with a second portion of the first particulate inhalant medicament to form a dry powder inhalation composition. In this aspect, the ratio by weight of the second particulate inhalant medicament to the carrier is less than the ratio by weight of the first particulate inhalant medicament to the carrier.

In one embodiment of this aspect, the first portion of the first medicament is less than half of the total quantity of the first medicament, while in yet other embodiments the first portion of the first medicament is less than 2 % weight by weight of the total amount of carrier.

While not wishing to be bound by theory, it is believed that a key aspect of the invention contributing to the uniformity of disposition, reliability and dose uniformityis that the first portion of the first medicament when mixed with the carrier creates a monolayer on the carrier. In an embodiment the first portion of the first medicament comprises a sufficient amount to create a monolayer of the first medicament on the particulate carrier.

The amount of medicament to form a close packed monolayer of first medicament on the carrier can be calculated using the following equation:

$$C^{\min} = 2\pi d \frac{(D+d)^2}{\sqrt{3}D^3}$$

where D and d are the volume median diameters (VMD) of the carrier and first medicament respectively. Thus for a carrier with a VMD of approximately 57.5 microns and a first medicament with a VMD of approximately 1.44 microns, $C^{\min} \approx 0.1\%$ (w/w). Thus, for example, in blending 2.15 grams of a first medicament with 47.72 grams of a particulate carrier, the first portion of first medicament to be added would be about 0.04772 grams. In some embodiments, the first portion of first medicament is added using a geometric mixing process.

Representative non-limiting examples of particulate carriers for use in the invention include, without limitation, lactose, glucose, or sodium starch glycolate particulates. In some embodiments, the particulate carrier is lactose. The particulate lactose is in some instances alpha lactose monohydrate. In general, the particle size of the lactose should be such that it can be entrained in an air stream but not deposited in the key target sites of the lung. Accordingly, in some embodiments, lactose with a mean particle size of less than 40 $\mu$m is excluded. Particle size is determined using laser light scattering (Sympatec GmbH, Claasthal-Zellerfeld, Germany). The carrier particles have a VMD of from about 50 to about 250 $\mu$m. Within that range, the carrier particles of a given composition according to the invention may have a VMD of from about 50 to about 60 $\mu$m or from about 60 to about 90 $\mu$m or from about 90 to about 150 $\mu$m.

As used herein, the recitation of a numerical range for a variable is intended to convey that the invention may be practiced with the variable equal to any of the values within that range. Thus, for a variable that is inherently discrete, the variable can be equal to any integer value of the numerical range, including the end-points of the range. Similarly, for a variable that is inherently continuous, the variable can be equal to any real value of the numerical range, including the end-points of the range. As an example, a variable which is described as having values between 0 and 2, can be 0,1 or 2 for variables which are inherently discrete, and can be 0.0, 0.1, 0.01, 0.001, or any other real value for variables which are inherently continuous.

In the exemplified methods and compositions the first medicament is a steroid and the second medicament is a bronchodilator. One of skill in the art will appreciate that the discovery that mixing a carrier with two or more medicaments in the sequential fashion as detailed herein confers certain appealing properties to the resultant composition need not be limited to the exemplified active substances. Hence, in some embodiments, the first medicament is an anti-inflammatory. A steroid contemplated is budesonide. In some embodiments the second medicament is a bronchodilator, in particular a long acting bronchodilator, such as formoterol or a pharmaceutically acceptable salt thereof.

The proportion of first medicament to second medicament by mass will range from about 5:1 to about 100:1. In other embodiments, the proportion of second medicament to carrier will be in the range of from about 10:1 to about 1:10,000.

An aspect of the invention provides a dry powder inhalation composition prepared by a process (as described above) comprising the steps of mixing a carrier with a first portion of a first particulate inhalant medicament to form an first mixture; thereafter mixing the first mixture with a second particulate inhalant medicament to form a second mixture; and mixing the second mixture with a second portion of the first particulate inhalant medicament to form a dry powder inhalation composition. In this aspect, the ratio by weight of the second particulate inhalant medicament to the carrier is less than the ratio by weight of the first particulate inhalant medicament to the carrier.

In some embodiments of this aspect, the first medicament is budesonide while in other embodiments the second medicament is formeterol. In yet other embodiments, the second medicament is formoterol fumerate dihydrate.

The compositions according to the invention are optionally formulated in a pharmaceutically acceptable vehicle with any of the well-known pharmaceutically acceptable medically inert moiety such as carriers, including diluents, excipients, surfactants, and flavourings (see Remington's Pharmaceutical Sciences, 18th Ed., Gennaro, Mack Publishing Co., Easton, PA 1990 and Remington. The Science and Practice of Pharmacy, Lippincott, Williams & Wilkins, 1995). While the type of pharmaceutically acceptable carrier/vehicle employed in generating the compositions of the invention will vary depending upon the mode of administration of the composition to a mammal, generally pharmaceutically acceptable carriers are physiologically inert and non-toxic.

As used herein, "medicament" or "active ingredient" is meant to encompass active pharmaceuticals appropriate for inhalation therapy in dry powder form. Representative, non-limiting examples include bronchodilators (*e.g.*, epinephrine, metaproterenol, terbutaline, albuterol, and the like), anticholinergic agents (*e.g.*, ipratropium bromide), xanthines (*e.g.*, dyphylline, aminophylline), inhalant corticosteroids (*e.g.*, flunisolide, beclomethasone, budesonide, and the like), or $\beta$-2 adrenergic receptor agonists (*e.g.*, salmeterol and formoterol).

The medicament may be in any isomeric form or mixture of isomeric forms, for example a pure enantiomer, particularly the R, R-enantiomer, a mixture of enantiomers, a racemate or a mixture thereof (*e.g.*, formoterol). Pharmaceutically acceptable derivatives include pharmaceutically acceptable salts, in particular acid addition salts with inorganic acids such as hydrochloric acid, hydrobromic acid, sulphuric or phosphoric acid. The salt may also be with an organic acid such as acetic, succinic, maleic, furmaric, citric, tartaric, lactic or benzoic. The active ingredient and pharmaceutically acceptable derivatives thereof may exist in the form of a solvate, in particular a hydrate.

A form of active ingredient for use in the invention is formoterol fumarate, especially formoterol fumarate dihydrate, conveniently in its racemic form. Formoterol, salts and hydrates thereof and salt hydrates thereof as described above may be prepared by known methods, for example as described in U.S. Patent 3,994,974 or U.S. Patent 5,684,199.

The formulations of the compositions of the invention may conveniently be presented in unit dosage form and may be prepared by conventional pharmaceutical techniques. Such techniques include the step of bringing into association the compound of the invention and the pharmaceutically acceptable carrier(s), or an excipient. In general, the formulations are prepared by uniformly and intimately bringing into association the active ingredient with finely divided solid carriers, and then, if necessary, preparing discrete dosage units of the product.

The dry powder composition may be metered and filled into capsules, e.g., gelatin or hydroxypropyl methylcellulose capsules, such that the capsule contains a unit dose of active ingredient.

When the dry powder is in a capsule containing a unit dose of active ingredient, the total amount of composition will depend on the size of the capsules and the characteristics of the inhalation device with which the capsules are being used. However, representative characteristic total fill weights of dry powder per capsule are between 1 and 25 mg, e.g., 5, 10, 15 or 20 mg.

Alternatively, the dry powder composition according to the invention may be filled into the reservoir of a multidose dry powder inhaler (MDPI), for example of the kind illustrated in WO 92/10229.

Another aspect of the invention provides for a dry powder inhaler comprising the inhaler and a composition according to the invention.

Another aspect of the invention provides a method for the administration of a particulate medicament, comprising inhalation of a composition of the invention from a multidose dry powder inhaler.

In yet another aspect, the invention provides a method for the administration of a therapeutically effective amount of compositions prepared by the processes described herein, for the treatment of conditions responsive to the medicaments of choice. Non-limiting examples of conditions include chronic obstructive pulmonary disease, asthma, late phase allergic responses, or pulmonary inflammations.

The term "therapeutically effective amount" is used to denote treatments at dosages effective to achieve the therapeutic result sought. Furthermore, one of skill will appreciate that the therapeutically effective amount of the compositions of the invention may be lowered or increased by fine tuning and/or by administering more than one composition of the invention, or by administering a composition of the invention with another compound or composition. The invention therefore provides a method to tailor the administration/treatment to the particular exigencies specific to a given mammal.

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein.

## EXAMPLES

### Example 1

### Preparation of budesonide/formoterol/lactose blends 100:6 and 200:6 microgram budesonide/formoterol blends at 2.5 kilo scale

| Blend Strength | Lactose | Budesonide | Formoterol |
|---|---|---|---|
| 100:6 | 2354.25 grams | 137.5 grams | 8.25 grams |
| 200:6 | 2373.8 grams | 122.5 grams | 3.7 grams |

### Stage 1

A monolayer of budesonide was formed on the lactose crystals employing 0.5 % weight by weight of budesonide. The required amount of lactose and budesonide (see Table I) were dispensed into separate stainless steel containers. Half the lactose was placed into a stainless steel mixing container with a lid. A 4 litre container was used for 1 kilo/2 kilo batches and both 8 litre and 10 litres containers for 2.5 kilo/batches. Any aggregates of budesonide were broken up with a spatula and the active ingredient was gradually added with even distribution over the lactose bed. The remaining lactose was added into the mixing vessel. The mixing vessel was then placed on a TURBULA™ mixer (TURBULA™, Glen Creston, New Jersey, USA) for 10 minutes at 23 or 32 rpm.

### Stage 2

The formoterol was added to the pre-blend from stage 1. The required amount of formoterol (see Table I) was weighed into a stainless steel beaker. The formoterol was added into the mixing container after breaking up any agglomerates with a spatula. This was added a spatula full at a time ensuring even distribution over the blend. The container was then replaced on the TURBULA™ mixer for 40 minutes at 46 rpm.

### Stage 3

The rest of the budesonide was added to the blend. The budesonide was dispensed into a stainless steel beaker. Half the pre-blend from stage 2 was added into the 3-litre bowl of an aeromatic fielder pma 1 granulator, (Nivo Pharma Systems (Nivo Inc.) Columbia, Maryland, US). The budesonide was subsequently added in, carefully ensuring an even distribution around the bowl. The remaining pre-blend was added in. The powder was mixed for 15 minutes with a granulator speed of 1500 rpm and a chopper speed of 600 rpm. The blend was discharged from the mixer into a double polythene bag. The blend was poured into a 250 micron sieve assembly and sieved at amplitude 0.65 millimetres using the Retsch sieve shaker.

Ten samples from different spots of the blend were taken for homogeneity analysis for both budesonide and formoterol. All blends were found to contain drugs close to the targets with relative standard deviation (RSD) of drug content < 5% (Table 2).

### Table 2

| Homogeneity Results for Budesonide and Formoterol Blends. | | | | | | |
|---|---|---|---|---|---|---|
| Batch Number | Budesonide Concentration % w/w | | | Formoterol Concentration %w/w | | |
| | Target | Actual | % RSD | Target | Actual | % RSD |
| RD-01-020 | 4.90 | 4.9 | 1.8 | 0.148 | 0.152 | 2.9 |
| RD-01-021 | 5.5 | 5.3 | 2.2 | 0.330 | 0.335 | 4.1 |
| RD-01-022 | 4.90 | 4.8 | 1.3 | 0.148 | 0.151 | 2.2 |
| RD-01-023 | 5.5 | 5.3 | 2.0 | 0.330 | 0.336 | 3.2 |

After the blend was found to be homogeneous in drug contents, it was then filled into a IVAX™ multidose DPI (MDPI), a DPI devise based on that disclosed in WO92/10229.

The inhalers that contained the formulation were then tested for pharmaceutical performance under conditions specified in European Pharmacopoeia (2001) including uniformity of delivered dose and fine particle dose. The drug per actuation (DPA) was measured using a dose unit sampling unit in conjunction with a critical flow controller model TPK, high capacity pump and flowmeter (Copley Scientific, Nottingham, U.K.) while fine particle dose (FPD) and fine particle fraction (FPF) were measured using a 5-stage liquid impinger MSL also from Copley Scientific.

The compositions gave excellent dose uniformity and reliability with mean DPA close to label claim for both medicaments when used in association with the device of WO 92/10229, with a good proportion of fine particles of both drugs (Tables 3 & 4).

### Table 3

| Pharmaceutical Assessment Results for the blends for the delivery of 100 mcg budesonide (Bud) and 6 meg formoterol (EML) | | | | | | |
|---|---|---|---|---|---|---|
| Batch No. | Device 1 | | Device 2 | | Device 3 | |
| % FPF | BUD | EML | BUD | EML | BUD | EML |
| RD-01-021 | 49.5 | 34.5 | 49.5 | 35.0 | 49.0 | 36.0 |
| RD-01-023 | 50.5 | 38.5 | 52.5 | 39.0 | 51.0 | 37.5 |
| FPD $\mu$g | | | | | | |
| RD-01-021 | 54.9 | 2.4 | 52.3 | 2.3 | 52.4 | 2.4 |
| RD-01-023 | 54.6 | 2.5 | 55.8 | 2.5 | 55.7 | 2.5 |
| Mean DPA | | | | | | |
| RD-01-021 | 111.8 | 6.5 | 105.6 | 6.6 | 108.9 | 6.7 |
| RD-01-023 | 105.8 | 6.3 | 108.6 | 6.5 | 110.6 | 6.6 |

### Table 4

| Pharmaceutical Assessment Results for the blends for the delivery of 200 meg budesonide (Bad) and 6 meg formoterol (EML) | | | | | | |
|---|---|---|---|---|---|---|
| Batch No. | Device | | Device 2 | | Device 3 | |
| % FPF | BUD | EML | BUD | EML | BUD | EML |
| RD-01-020 | 51.5 | 38.0 | 52.0 | 38.0 | 48.0 | 33.5 |
| RD-01-022 | 49.0 | 35.5 | 52.5 | 37.5 | 47.0 | 34.0 |
| FPD $\mu$g | | | | | | |
| RD-01-020 | 111.2 | 2.4 | 113.5 | 2.5 | 99.7 | 2.1 |
| RD-01-022 | 97.0 | 2.2 | 103.0 | 2.2 | 95.9 | 2.1 |
| Mean DPA | | | | | | |
| RD-01-020 | 212.0 | 6.3 | 225.6 | 6.7 | 216.3 | 6.5 |
| RD-01-022 | 217.2 | 6.5 | 206.2 | 6.1 | 206.7 | 6.2 |

**Equivalents**

While the claimed invention has been described in detail and with reference to specific embodiments thereof, it will be apparent to one of ordinary skill in the art that various changes and modifications can be made to the claimed invention without departing from the scope thereof. Thus, for example, those skilled in the art will recognize, or be able to ascertain, using no more than routine experimentation, numerous equivalents to the specific substances and procedures described herein. Such equivalents are considered to be within the scope of this invention, and are covered by the following claims.

**Claims**

1. A method of preparing a dry powder inhalation composition comprising the steps of:

   (a) mixing a carrier with a first portion of a first particulate inhalant medicament to form a first mixture;
   (b) mixing said first mixture with a second particulate inhalant medicament to form a second mixture; and
   (c) mixing said second mixture with a second portion of the first particulate inhalant medicament to form a dry powder inhalation composition,

   wherein, in the dry powder inhalation composition from step (c) the ratio by weight of the second particulate inhalant medicament to the carrier is less than the ratio by weight of the first particulate inhalant medicament to the carrier, wherein the ratio of the first particulate inhalant medicament to the second particulate inhalant medicament by weight is from 5:1 to 100:1.

2. The method according to claim 1, wherein the first portion of the first particulate inhalant medicament is less than half weight by weight of the total amount of the first particulate inhalant medicament in the dry powder inhalation composition.

3. The method according to claim 1 or claim 2, wherein the first portion of first particulate inhalant medicament is less than 2% weight by weight of the total amount of carrier.

4. The method according to any one of claims 1 to 3, wherein the first portion of the first particulate inhalant medicament is sufficient to create a monolayer of the first particulate inhalant medicament on the carrier.

5. The method according to any one of claims 1 to 4, wherein the carrier is selected from lactose, glucose or sodium starch glycolate.

6. The method according to any one of claims 1 to 5, wherein the carrier is lactose.

7. The method according to any one of claims 1 to 6, wherein the first particulate inhalant medicament is an anti-inflammatory steroid or a pharmaceutically acceptable derivative thereof.

8. The method according to any one of claims 1 to 7 where the first particulate inhalant medicament is budesonide or a pharmaceutically acceptable derivative thereof.

9. The method according to any one of claims 1 to 8, where the second particulate inhalant medicament is a bronchoditator or a pharmaceutically acceptable derivative thereof.

10. The method according to any one of claims 1 to 9, where the second particulate inhalant medicament is formoterol or a pharmaceutically acceptable derivative thereof.

11. A method of preparing a multidose dry powder inhaler (MDPI), which comprises preparing a dry powder inhalation composition by a method as claimed in any one of claims 1 to 11, and then filling the composition into the reservoir of a multidose dry powder inhaler.

**Patentansprüche**

1. Eine Methode zur Herstellung einer Trockenpulver-Formulierung zur Inhalation bestehend aus den folgenden Schritten:

   (a) Mischen eines Trägersstoffs mit einer ersten Portion eines ersten Partikelinhalationsmedikaments zur Bildung eines ersten Gemischs;
   (b) Mischen des genannten ersten Gemischs mit einem zweiten Partikelinhalationsmedikament zur Bildung eines zweiten Gemischs, und
   (c) Mischen des genannten zweiten Gemischs mit einer zweiten Portion des ersten Partikelinhalationsmedikaments zur Bildung einer Trockenpulver-Formulierung zur Inhalation,

   wobei in der Trockenpulver-Formulierung zur Inhalation aus Schritt (c) das Gewichtsverhältnis des zweiten Partikelinhalationsmedikaments zum Trägerstoff geringer ist als das Gewichtsverhältnis des ersten Partikelinhalationsmedikaments zum Trägerstoff,
   wobei das Gewichtsverhältnis des ersten Partikelinhalationsmedikaments zum zweiten Partikelinhalationsmedikament 5:1 bis 100:1 beträgt.

2. Die Methode nach Anspruch 1, **dadurch gekennzeichnet, dass** das Gewicht der ersten Portion des ersten Partikelinhalationsmedikaments weniger als das halbe Gewicht der gesamten Menge des ersten Partikelinhalationsmedikaments in der Trockenpulver-Formulierung zur Inhalation beträgt.

3. Die Methode nach Anspruch 1 oder Anspruch 2, **dadurch gekennzeichnet, dass** das Gewicht der ersten Portion des ersten Partikelinhalationsmedikaments weniger als 2 % des Gewichts der gesamten Menge des Trägerstoffs beträgt.

4. Die Methode nach einem der Ansprüche 1 bis 3, **dadurch gekennzeichnet, dass** die erste Portion des ersten Partikelinhalationsmedikaments ausreichend ist zur Herstellung einer Monolage des ersten Partikelinhalationsmedikaments auf dem Trägerstoff.

5. Die Methode nach einem der Ansprüche 1 bis 4, **dadurch gekennzeichnet, dass** der Trägerstoff aus Laktose, Glukose oder Natriumstärkeglykolat gewählt wird.

6. Die Methode nach einem der Ansprüche 1 bis 5, **dadurch gekennzeichnet, dass** der Trägerstoff Laktose ist.

7. Die Methode nach einem der Ansprüche 1 bis 6, **dadurch gekennzeichnet, dass** das erste Partikelinhalationsmedikament ein entzündungshemmendes Steroid oder eines seiner pharmazeutisch akzeptablen Derivate ist.

8. Die Methode nach einem der Ansprüche 1 bis 7, **dadurch gekennzeichnet, dass** das erste Partikelinhalationsmedikament Budesonid oder eines seiner pharmazeutisch akzeptablen Derivate ist.

9. Die Methode nach einem der Ansprüche 1 bis 8, **dadurch gekennzeichnet, dass** das zweite Partikelinhalationsmedikament ein Bronchodilatator oder eines seiner pharmazeutisch akzeptablen Derivate ist.

10. Die Methode nach einem der Ansprüche 1 bis 9, **dadurch gekennzeichnet, dass** das zweite Partikelinhalationsmedikament Formoterol oder eines seiner pharmazeutisch akzeptablen Derivate ist.

11. Eine Methode zur Herstellung eines Mehrfachdosis-Trockenpulverinhalators (MDPI), die die Herstellung einer Trockenpulver-Formulierung zur Inhalation mittels einer Methode nach einem der Ansprüche 1 bis 11 sowie das anschließende Abfüllen der Formulierung in den Behälter eines Mehrfachdosis-Trockenpulverinhalators umfasst.

**Revendications**

1. Une méthode de préparation d'une composition pour inhalation à base de poudre sèche comportant les étapes suivantes :

   (a) mélange d'un transporteur avec une première portion d'un premier médicament particulaire pour inhalation afin de former un premier mélange ;

(b) mélange dudit premier mélange avec un second médicament particulaire pour inhalation afin de former un second mélange ; et

(c) mélange dudit second mélange avec une seconde portion du premier médicament particulaire pour inhalation afin de former une composition pour inhalation à base de poudre sèche,

au sein de laquelle, dans la composition pour inhalation à base de poudre sèchs issue de l'étape (c), le rapport en poids du second médicament particulaire pour inhalation par rapport au transporteur soit inférieur au rapport en poids du premier médicament particulaire pour inhalation par rapport au transporteur,

au sein de laquelle le rapport en poids du premier médicament particulaire pour inhalation par rapport au second médicament particulaire pour inhalation soit, en poids, de l'ordre de 5:1 à 100:1.

2. Selon la revendication 1, la méthode au sein de laquelle la première portion du premier médicament particulaire pour inhalation est inférieure en poids à la moitié du poids de la quantité totale du premier médicament particulaire pour inhalation dans la composition pour inhalation à base de poudre sèche.

3. Selon la revendication 1 ou 2, la méthode, au sein de laquelle la première portion du premier médicament particulaire pour inhalation est inférieure en poids à 2% de la quantité totale de transporteur.

4. Selon les revendications 1 à 3, au sein desquelles la première portion du premier médicament particulaire pour inhalation est suffisante pour créer une monocouche du premier médicament particulaire pour inhalation sur le transporteur.

5. Selon n'importe laquelle des revendications 1 à 4, la méthode au sein de laquelle le transporteur est sélectionné parmi le lactose, le glucose ou le glycolate d'amidon sodique.

6. Selon n'importe laquelle des revendications 1 à 5, la méthode au sein de laquelle le lactose est utilisé comme transporteur.

7. Selon n'importe laquelle des revendications 1 à 6, la méthode au sein de laquelle le premier médicament particulaire pour inhalation est un stéroïde anti-inflammatoire ou un dérivé pharmaceutiquement acceptable de celui-ci.

8. Selon n'importe laquelle des revendications 1 à 7, la méthode au sein de laquelle le budésonide ou un dérivé pharmaceutiquement acceptable de celui-ci est utilisé comme premier médicament particulaire pour inhalation.

9. Selon n'importe laquelle des revendications 1 à 8, la méthode au sein de laquelle le second médicament particulaire pour inhalation est un bronchodilatateur ou un dérivé pharmaceutiquement acceptable de celui-ci.

10. Selon n'importe laquelle des revendications 1 à 9, au sein de laquelle le formotérol ou un dérivé pharmaceutiquement acceptable de celui-ci est utilisé comme second medicament particulaire pour inhalation.

11. Une méthode de préparation d'un inhalateur multidose de poudre sèche (MDPI), qui comprend la préparation d'une composition de poudres sèches pour inhalation par une méthode telle que définie dans n'importe laquelle des revendications 1 à 11 et le remplissage subséquent de la composition dans le réservoir d'un inhalateur multidose de poudres sèches.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0170198 A
- US 3994974 A
- US 5684199 A
- WO 9210229 A

### Non-patent literature cited in the description

- **GOODMAN ; GILMAN'S.** The Pharmacological Basis of Therapeutics. McGraw Hill Companies Inc, 2001
- Remington's Pharmaceutical Sciences. Mack Publishing Co, 1990
- Remington. The Science and Practice of Pharmacy. Lippincott, Williams & Wilkins, 1995